Europäisches Patentamt

European Patent Office

Office européen des brevets

(19)

(11) **EP 1 466 966 A1**

(12) **DEMANDE DE BREVET EUROPEEN**

(43) Date de publication:
**13.10.2004 Bulletin 2004/42**

(51) Int Cl.7: **C12M 1/33**, C12M 3/08

(21) Numéro de dépôt: **04012952.0**

(22) Date de dépôt: **03.04.2000**

(84) Etats contractants désignés:
**AT BE CH CY DE DK ES FI FR GB GR IE IT LI LU MC NL PT SE**

(30) Priorité: **01.04.1999 FR 9904289**

(62) Numéro(s) de document de la (des) demande(s) initiale(s) en application de l'article 76 CBE:
**00915279.4 / 1 165 748**

(71) Demandeur: **Biomerieux S.A.**
**69280 Marcy l'Etoile (FR)**

(72) Inventeurs:
• **Colin, Bruno**
**69280 Marcy l'Etoile (FR)**
• **Cleuziat, Philippe**
**69003 Lyon (FR)**

• **Broyer, Patrick**
**69100 Villeurbanne (FR)**
• **Mabilat, Claude**
**69650 Saint-Germain au Mont-d'Or (FR)**
• **Incardona, Sandra**
**69003 Lyon (FR)**

(74) Mandataire: **Macquet, Christophe**
**Murgitroyd & Company,**
**Immeuble Atlantis,**
**55. allée Pierre Ziller**
**06560 Valbonne Sophia Antipolis (FR)**

Remarques:
Cette demande a été déposée le 01 - 06 - 2004 comme demande divisionnaire de la demande mentionnée sous le code INID 62.

(54) **Appareil et procédé de lyse par ultrasons de cellules biologiques**

(57)     La présente invention concerne un appareil, comprenant une sonotrode (2) destinée à générer des ultrasons de plus ou moins forte puissance dans au moins un échantillon biologique (5) contenant des cellules à lyser, échantillon (5) qui est contenu dans au moins un récipient (4 ou 10) adapté à cette usage. Elle concerne également un appareil comprenant au moins deux sonotrodes et un procédé de lyse par ultrasons d'un échantillon biologique (5) contenu dans un récipient (4), qui utilise au moins une telle sonotrode (2).

L'appareil se caractérise par le fait que la sonotrode (2) est directement en contact avec le ou les récipients (4). Le procédé consiste à :

- positionner le récipient (4) au contact direct de la sonotrode (2),
- activer ladite sonotrode (2) pendant un temps suffisant pour lyser les cellules contenues dans l'échantillon (5) en conservant ou les ADN et/ou ARN libérés pour permettre leur utilisation ultérieure, par exemple dans une étape d'amplification.

L'invention trouve une application préférentielle dans le domaine de la biologie moléculaire.

**Fig. 2**

## Description

La présente invention concerne un appareil et un procédé pour générer des ultrasons de plus ou moins forte puissance dans un récipient au niveau d'un échantillon à lyser avec ou sans présence de billes de verres en son sein. La gamme de fréquence ultrasonore varie de 20 à 50 kiloHertz (kHz) environ.

L'état de la technique est essentiellement constitué de deux techniques, qui utilisent des ultrasons en biologie comme méthode de lyse des micro-organismes.

La première technique consiste en la sonification directe en plongeant la sonotrode dans l'échantillon liquide à lyser. Le principe utilisé dans ce cas consiste à mettre en contact la sonotrode directement avec le liquide et à générer en continu ou en mode impulsionnel des ultrasons de forte puissance induisant une cavitation très intense dans le milieu. Les sonotrodes utilisées sont généralement de forte puissance (100 à 1000 Watts (W)) et permettent de lyser des échantillons très divers réputés difficiles à lyser en très peu de temps (de l'ordre de la minute).

Toutefois, deux inconvénients majeurs limitent l'utilisation de cette technique.

Tout d'abord, le phénomène de cavitation étant un phénomène très difficilement contrôlable, le pourcentage de lyse sur un nombre important d'essais ne sera donc pas reproductible.

De plus, cette cavitation très intense induit un clivage intense non contrôlé des acides nucléiques, ce qui peut être problématique lorsque l'utilisateur cherche à lyser, à détecter et à amplifier quelques dizaines ou centaines de micro-organismes par millilitre (concentration très faible).

Enfin, la sonotrode étant directement en contact avec l'échantillon, cette technique n'est pas utilisable de manière automatisée sur des prélèvements de patients, à moins de prévoir une étape de nettoyage de ladite sonotrode, étape longue et coûteuse à mettre en oeuvre dans un automate.

D'autres inconvénients en rapport aux problèmes ci-dessus décrits existent. Ainsi, la cavitation entraîne la condensation d'une partie de l'échantillon, ce qui réduit encore la finesse de l'analyse réalisée. Enfin, si la puissance acoustique est trop importante, l'échauffement excessif du tube peut aboutir à des dégradations, voire à la fonte dudit tube.

*La seconde technique consiste en la sonication en bain, qui est bien exposée dans le brevet US-5,374,522. Le principe consiste à utiliser un bain de nettoyage à ultrasons rempli d'eau, et dans lequel baigne la partie inférieure des tubes contenant les échantillons à lyser. Les échantillons sont soumis à un champ ultrasonore, en général constant, pendant une durée d'environ 15 minutes (mn). Les billes présentes dans les tubes sont alors soumises à une forte agitation entraînant des chocs et des contraintes de cisaillement suffisants pour lyser les micro-organismes. Contrairement à la première technique ci-dessus exposée, cette seconde technique nécessite l'utilisation de billes de verre pour obtenir une efficacité de lyse satisfaisante. Comparativement à la technique précédente d'insonication directe, cette technique permet de résoudre certains des problèmes évoqués précédemment.*

*Ainsi, les échantillons étant contenus dans un tube clos, il n'y a pas de contamination de l'élément vibratoire puisque les ultrasons sont transmis via l'eau (qui est un excellent couplant ultrasonore) du fond de la cuve jusqu'à l'intérieur du tube échantillon.*

*De plus, la densité volumique d'énergie (exprimée en Watt par millilitre (*W/ml*)) libérée dans le milieu étant environ 300 fois plus faible pour la même fréquence, le phénomène de cavitation est moins important.*

L'inconvénient majeur de cette technique réside dans sa difficulté à être automatisée étant donné la gestion du liquide (remplissage et vidange de la cuve), le dégazage nécessaire avant la sonication et la gestion de tubes humides après sonication, qui gouttent, ce qui nuit à la propreté.

D'autres inconvénients existent. Ainsi, les résultats ne sont pas reproductibles puisque la puissance acoustique n'est pas équivalente dans toutes les zones du récipient recevant le bain pour la sonification. Ceci est dû à la construction même des bains à ultrasons qui comportent, sous la cuve, un ou plusieurs transducteurs. Or le champ ultrasonore rayonne par l'intermédiaire d'un transducteur, ce qui n'est pas homogène dans l'espace. Par conséquent, le degré de lyse des micro-organismes ou le clivage des acides nucléiques est extrêmement hétérogène d'un échantillon à un autre, et n'est pas contrôlable.

Il existe toutefois des techniques de sonication par contact direct de la sonotrode avec le contenant de l'échantillon biologique à lyser. C'est par exemple le cas de la demande de brevet EP-A-0.337.690.

Toutefois, le contact entre le récipient et la sonotrode est constitué par une surface plane qui ne permet pas d'assurer une lyse homogène dans tout l'échantillon à lyser. De tels dispositifs ne répondent pas aux exigences de qualité de lysat nécessaires à la libération d'acides nucléiques. La seule solution est alors d'augmenter considérablement la durée de cette lyse, au moins le double de temps, avec les risques de dénaturation, voire de destruction, des acides nucléiques les plus exposés à cette sonication hétérogène.

Un comparatif de la densité d'énergie (W/ml) permet de comprendre la grande différence entre les deux techniques de l'état de la technique présentées ci-dessus. La densité d'énergie peut être définie par l'intégrale dans le temps de la puissance délivrée à l'échantillon ramenée au volume de l'échantillon. Cette densité peut être évaluée expérimentalement, en mesurant l'élévation de température obtenue dans un temps donné pour ces deux techniques.

$$E = \int_0^T \frac{W}{V}\, dt$$

avec

E : densité volumique d'énergie.
W : puissance (W)
V : Volume d'échantillon (ml)

Les mesures de température effectuées dans les tubes après, 15 mn de sonication en bain, permettent d'avoir une évaluation de la puissance délivrée à l'échantillon :

$T_{0mn}$ = 21°C
$T_{15mn}$ = 40,6°C

soit un $\Delta t$ = 19,6°C et DW = $\Delta t$ x 0,3ml = 19,6 x 0,3 = 5,88 cal/15mn
donc

$$Wt = (5{,}88 \times 4{,}18) / (60 \times 15) = 0{,}027\ W$$

La densité d'énergie D (en W/ml) dans un bain à ultrasons de type sonification en bain est :

$$D = 0{,}027 / 0.3 = 0{,}09\ W/ml$$

On constate bien que la densité d'énergie est environ trois cents fois plus faible que celle obtenue avec une sonotrode directement plongée dans le milieu, puisque, d'après la littérature, la densité d'énergie est généralement d'au moins 30 W. Les informations données dans le document US-A-5,374,522 sur la sonication en bain confirme cette valeur.

Conformément à la présente invention, l'appareil proposé permet de répondre à l'ensemble des problèmes ci-dessus exposés en contrôlant le clivage des acides nucléiques, tout en préservant l'intégrité du tube contenant l'échantillon. La propreté de l'ensemble est parfaite puisqu'il n'y a aucun contact entre l'extérieur du tube et un quelconque liquide, le contenu dudit tube étant complètement isolé de l'extérieur. Cet isolement interdit les projections vers l'extérieur lorsque le tube subit la sonification, et limite considérablement la condensation et l'évaporation.

A cet effet, la présente invention concerne un appareil, comprenant une sonotrode destinée à générer des ultrasons de plus ou moins forte puissance dans au moins un échantillon biologique contenant des cellules à lyser, échantillon qui est contenu dans au moins un récipient adapté à cette usage, la sonotrode est directement en contact avec le ou les récipients, contenant l'échantillon à lyser, en l'absence de fluide entre lesdites surfaces en contact entre la sonotrode et le récipient, caractérisé par le fait que la sonotrode possède une surface active qui épouse la forme de tout ou partie de chaque récipient contenant l'échantillon à lyser ; la surface active de la sonotrode en contact avec le récipient est de forme concave.

Dans un autre mode de réalisation, la surface active de la sonotrode en contact avec le récipient est de forme convexe.

Lorsque la surface active est convexe, la surface du récipient, qui coopère avec la sonotrode, est flexible de sorte qu'elle vient épouser la surface active de ladite sonotrode.

Dans tous les cas de figure, la sonotrode coopère avec au moins une bille de verre située dans l'échantillon contenu dans un récipient.

Chaque bille a un diamètre de 90 à 150 et préférentiellement de 100 micromètres ($\mu$m) dans le cas d'une lyse de bactéries, et un diamètre de 150 à 1500 et préférentiellement de 500 $\mu$m dans le cas d'une lyse de levures. Ces valeurs ne sont pas théoriques et ont déjà fait l'objet de recherches poussées réalisées par la demanderesse, qui ont abouties au dépôt d'une demande de brevet WO9915621. L'originalité de ce principe réside dans le fait que ces valeurs sont également applicables à la technique de sonication directe.

Selon une variante de réalisation particulièrement intéressante, l'appareil comporte au moins deux sonotrodes.

De plus, chaque récipient est en contact physique avec la ou les sonotrodes par un moyen de mise sous pression.

Chaque sonotrode possède une fréquence d'émission des ultrasons comprise entre 20 et 50 kHz, plus précisément entre 30 et 40 kHz et préférentiellement de sensiblement 35 kHz.

La présente invention concerne également un procédé de lyse par ultrasons de cellules contenues dans un échantillon biologique présent dans un récipient, qui utilise au moins une sonotrode. Selon une première mise en oeuvre, le procédé se caractérise en ce qu'il consiste à :

- positionner le récipient au contact direct avec la surface active de la ou des sonotrodes, et
- activer ladite ou lesdites sonotrodes pendant un temps suffisant pour lyser les cellules contenues dans l'échantillon en conservant les ADN et/ou ARN libérés pour permettre leur utilisation ultérieure, par exemple dans une étape d'amplification.

Selon une seconde mise en oeuvre, le procédé se caractérise en ce qu'il consiste à :

- positionner le récipient au contact direct avec la surface active de la ou des sonotrodes,
- activer ladite ou lesdites sonotrodes pendant un temps suffisant pour lyser les cellules contenues dans l'échantillon en clivant les ADN et/ou ARN libérés, tout en permettant leur utilisation ultérieure, par exemple dans une étape d'amplification.

Selon une variante de réalisation des deux mises en oeuvre précédentes, et préalablement à l'activation de la ou des sonotrodes, le récipient est pressé à l'encontre de la surface active de ladite ou desdites sonotrodes.

L'activation de chaque sonotrode s'effectue dans les conditions suivantes :

- durée de la sonication 10 à 15 minutes,
- rapport cyclique compris entre 40 et 60 % et préférentiellement 50 %, et
- puissance 10 à 30 W.

Selon un mode préférentiel de mise en oeuvre du procédé, l'activation de chaque sonotrode consiste en une série d'impulsions dont la durée est comprise entre 5 et 20 secondes, et préférentiellement entre 10 et 15 secondes.

Les figures ci-jointes sont données à titre d'exemple explicatif et n'ont aucun caractère limitatif. Elles permettront de mieux comprendre l'invention.

La figure 1 représente une sonotrode de 20kHz dans une configuration d'utilisation avec un tube.

La figure 2 représente une sonotrode de 35kHz , selon l'invention, dans une configuration d'utilisation avec un tube avant que celui-ci ne soit mis en place pour la sonication.

La figure 3 représente une sonotrode de 35kHz, selon la figure 2, mais utilisable avec douze tubes.

La figure 4 représente une carte en position horizontale dont le contenu subit une lyse par sonication directe selon l'invention.

La figure 5 représente une carte en position verticale dont le contenu subit une lyse par sonication directe selon l'invention.

La figure 6 représente une vue plus précise de la figure 4 montrant le comportement du liquide et des billes de verre durant la sonication directe.

La présente invention concerne un dispositif de lyse de micro-organismes par ultrasons, sans utilisation de liquide de couplage entre l'émetteur ultrasonore et l'échantillon biologique. Ce dispositif est utilisable en vue de la libération d'acides nucléiques en contrôlant leur clivage et en maximisant la quantité de matériel génétique libéré.

Un autre objet de la présente invention consiste en un nouveau principe de sonication dont l'originalité provient du fait que la transduction s'effectue directement, également appelée sonication directe. Elle consiste à coupler directement le ou les tubes ou tout autre récipient adapté, contenant l'échantillon à lyser, sur une sonotrode adaptée à la forme du tube ou des tubes. Dans ce cas, il n'y a donc plus de liquide de couplage.

Encore un autre objet de la présente invention consiste à trouver des paramètres de sonication permettant de lyser de manière efficace l'échantillon avec un clivage contrôlé des acides nucléiques libérés dans le milieu.

## I) DIFFERENTS MODES DE REALISATION DE L'APPAREIL DE LYSE :

### A - Premier mode de réalisation :

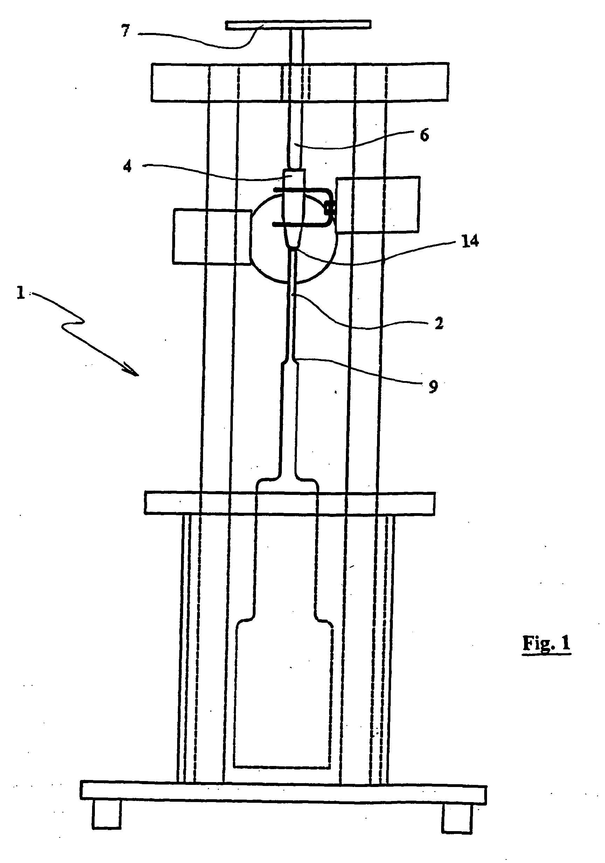

Un premier appareil de lyse 1 a été évalué. Ce type d'appareil 1 est bien représenté en figure 1.

L'appareil 1 comporte une sonotrode 2 modèle V1A fonctionnant à 20 kHz dont le diamètre est de 4 millimètres (mm) à son extrémité. Le générateur 3 utilisé pour exciter la sonotrode 2 est un générateur 3 de 300W (BioBlock, Type VibraCell, Modèle 72401) pouvant fonctionner en mode continu ou impulsionnel. La puissance de sortie ainsi que le rapport cyclique des impulsions (10 à 90 %) sont réglables. Le rapport cyclique est défini comme suit :

$$\text{Rapport cyclique (\%) = Ton / (Ton + Toff)}$$

avec Ton et Toff définissant un régime impulsionnel, dans lequel Ton est la durée de génération d'ultrasons et Toff la durée de pause entre deux Ton.

Le tube 4, contenant un échantillon 5 à lyser, fait office de récipient, est positionné sur l'extrémité de cette sonde 2 et est maintenu par mise sous pression à l'aide du plateau supérieur 6. Un poids 7 posé sur le plateau 6 permet d'avoir un couplage plus ou moins important entre l'extrémité de la sonotrode 2 et le tube 4, en fonction de la masse du poids 7. Les tubes 4 utilisés pour tous les essais sont de type Cryo Tube NUNC 1,8 ml avec un fond hémisphérique en forme sensiblement de U. D'autres formes ou marques de tubes peuvent également être utilisées.

Une fois l'ensemble mis en place, le tube 4 est soumis aux ultrasons pendant une durée variable généralement comprise entre 1 et 15 minutes (mn).

Les essais ont montré que des réglages entre les différents paramètres (puissance, durée, rapport cyclique, quantité de billes de verre, poids de couplage sur le plateau... ) permettent d'obtenir une lyse de micro-organismes satisfaisante sans dégradation du tube et avec une conservation des acides nucléiques variable suivant les paramètres choisis.

Le calcul de la densité de puissance transmise au tube 4 permet d'évaluer la dégradation éventuellement des tubes 4 ainsi que l'importance de la cavitation.

$$\text{Densité de puissance } Dp\ (W/cm^2) = Pe\ (W) \times \eta\ /\ Sc\ (cm^2)$$

avec,

Pe :     Puissance électrique fournie par le générateur (en W),
$\eta$ :     Coefficient de conversion de la Puissance Electrique en Puissance Acoutique (sans dimension)
Sc :     Surface de contact Sonotrode-Tube (en $cm^2$)

La puissance électrique incidente est généralement de 20 W (15 à 35 W testé), le rendement de conversion électro-acoustique peut être évalué à 80%, soit dans le cas de cet appareil 1, $Dp = 20 \times 0{,}8\ /\ (3{,}14 \times 0{,}2^2) = 127\ W/cm^2$.

**B - Deuxième mode de réalisation :**

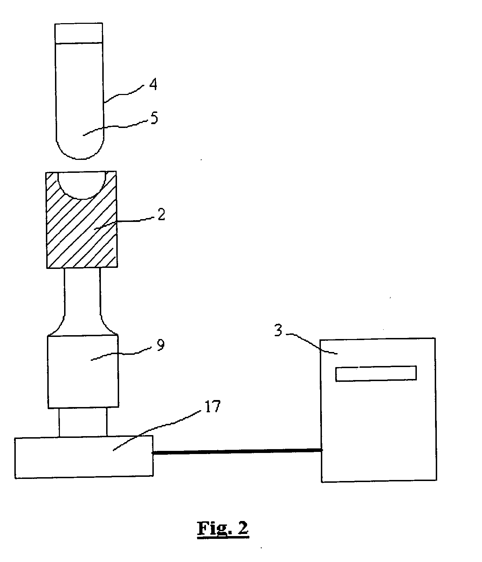

Un second appareil de lyse 1 a été réalisé, il concerne un système de sonication directe à 35kHz, au lieu de 20 kHz. Cet appareil 1 est représenté à la figure 2. Même si la structure externe est identique pour ces deux sonotrodes respectivement de 20 et 35 kHz, la sonotrode de 35 kHz possède de nombreux avantages.

Premièrement, il permet, à puissance équivalente, de diminuer l'importance du phénomène de cavitation. Le seuil de cavitation, c'est-à-dire la limite de puissance entre deux domaines avec ou sans cavitation, augmente sensiblement de manière parabolique en fonction de la fréquence. Ainsi, à puissance acoustique absorbée équivalente, la cavitation est un peu moins importante à 35 kHz qu'à 20 kHz.

Deuxièmement, il minimise les projections de l'échantillon (gênantes pour la récupération du lysat) constatées avec la sonotrode 20 kHz dues à sa forte amplitude vibratoire.

Enfin et troisièmement, il réduit l'encombrement du système puisque la longueur des éléments, composant la sonotrode 2, diminue lorsque la fréquence augmente.

Cette sonotrode 2, fonctionnant à 35kHz, est alimentée par un générateur 3 de 500W type MW 1000 GSIP (société SODEVA). Le même plateau 6 permet de mettre le tube 4 sous pression de manière à ajuster le couplage entre ledit tube 4 et la sonotrode 2.

Quel que soit la sonotrode 2 utilisée, 20 ou 35 kHz, l'utilisation d'un booster 9 permet, suivant son rapport de section et la position dans laquelle il est placé, d'amplifier ou d'atténuer l'amplitude vibratoire à l'extrémité de la sonde 2. Cette amplification ou atténuation est directement proportionnelle au rapport des dimensions du tube 4 ou de tout autre récipient utilisé. Ainsi par exemple, une réduction de diamètre du tube de 15 à 12 mm permet de multiplier l'amplitude des vibrations par 15/12 = 1,25.

En fait, le booster 9 est constitué par un cylindre métallique, qui est dans un même matériau que le reste de la sonde, généralement du titane ou de l'aluminium. Celui-ci comporte un changement de diamètre progressif dans sa partie médiane. La longueur dudit booster 9 correspond à la demi-longueur d'onde acoustique pour des questions d'adaptation acoustique.

Le rôle du booster 9 est d'amplifier l'amplitude de vibration à l'extrémité de la sonde et donc par conséquent la puissance acoustique délivrée au milieu. Cette amplification vibratoire est directement proportionnelle au rapport des deux diamètres du booster 9.

La figure 2 décrit les différents éléments de la sonde 2, le support mécanique et le plateau de mise sous pression 6 ne sont pas représentés.

Pour estimer la densité de puissance, on calcule la surface de contact d'une hémisphère,

$$Sc = 2\pi R^2 = 2 \times 3.14 \times 0.6^2 = 2.26 \ cm^2$$

soit en prenant 20 W électrique incident et un coefficient de conversion de 80%, on obtient dans le cas du deuxième appareil 1 :

$$Dp = 20 \times 0.8 \ / \ 2.26 = 7.1 \ W/cm^2$$

c'est-à-dire une densité de puissance dix-huit fois plus faible que pour le premier appareil 1.

Cette densité de puissance beaucoup plus faible permet de soumettre le tube 4 à une puissance plus grande sans dégradation de celui-ci.

### C - Troisième mode de réalisation :

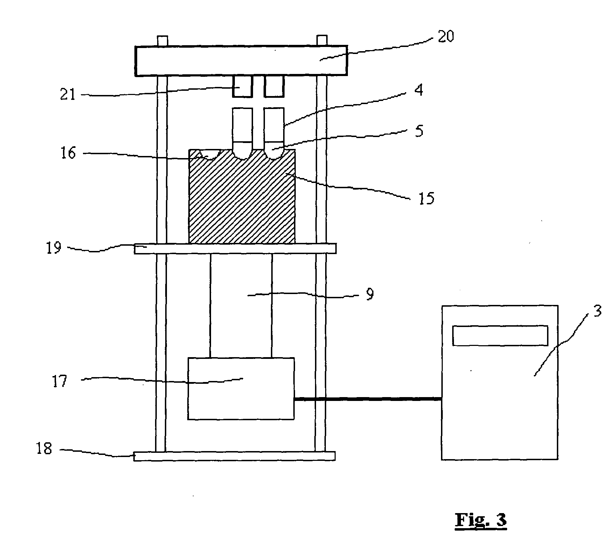

Le procédé peut également être mis en oeuvre sur plusieurs tubes 4. Il est donc possible d'avoir un troisième mode de réalisation de l'appareil 1, qui permet de lyser simultanément le contenu d'au moins deux tubes, comme cela est représenté en figure 3. La fréquence est identique et la forme du couplage est semblable au deuxième appareil 1 de 35 kHz, dont la surface active est hémisphérique, comme cela sera décrit par la suite. Un système à ressort permet d'ajuster individuellement la pression exercée sur chaque tube 4 afin d'avoir une force de couplage identique pour toutes les positions de la sonotrode 2.

### II) DIFFERENTS MODES DE REALISATION DU RECIPIENT CONTENANT L'ECHANTILLON A TRAITER :

### A - Premier mode de réalisation :

Le premier mode de réalisation est bien entendu constitué par un tube 4, tout à fait classique, comme cela est bien représenté aux figures 1 à 3. Néanmoins la forme des tubes 4 représentés sur ces figures n'est pas limitative, et de nombreuses autres formes, plus ou moins évasées, avec ou sans au moins une zone tronconiques qui est inversée ou non, etc., peuvent être utilisé par l'invention.

Dans ce cas, le premier appareil de lyse 1 est moins efficace que le second. Ainsi, avec le second appareil de lyse, le couplage entre la sonotrode 2 et le tube 4 est amélioré par la réalisation d'une extrémité concave adaptée à la forme en U dudit tube 4. La puissance acoustique étant transmise à travers une surface de contact plus grande, un domaine plus large de puissance pourra donc être utilisé sans dégrader le tube 4 du fait d'une dissipation par effet Joule excessive due à une densité de puissance trop élevée, la surface de contact étant trop réduite.

De plus, la surface active 8 de la sonotrode 2 étant en forme de U, cette forme permet d'avoir un positionnement parfait est constant du récipient 4 par rapport à la sonotrode 2, ce qui autorise une meilleure reproductibilité d'un essai à l'autre. Avec le premier appareil, cette reproductibilité des résultats était plus difficile à contrôler.

De plus, cette forme permet également de répartir de manière beaucoup plus homogène les ultrasons et donc d'obtenir une meilleure agitation dans le récipient ou tube 4. Il est possible et même préférentiel d'avoir, dans ledit récipient 4, des billes 9. Là encore le résultat de la lyse est reproductible.

### B - Deuxième mode de réalisation :

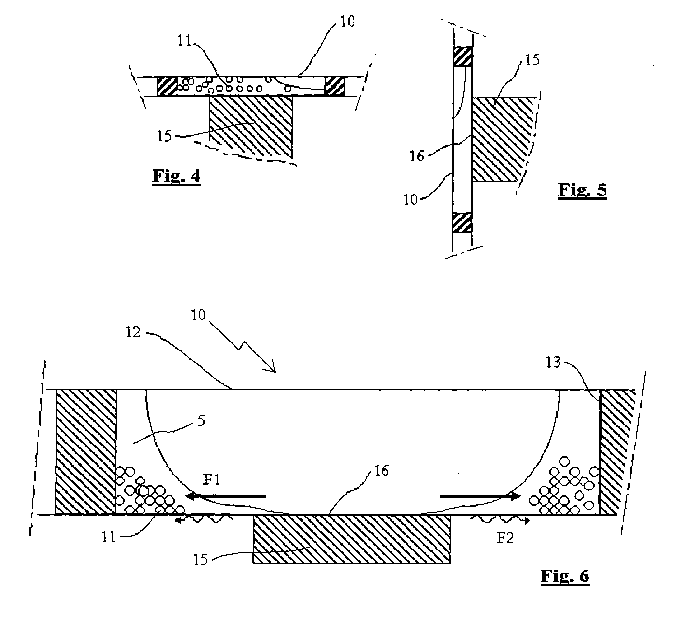

Ce mode de réalisation est représenté sur les figures 4 à 6. Il s'agit d'une carte 10 de matière plastique, qui comporte au moins un trou transversal ou puits 13, tranversant de part en part la carte 10. Le puits 13 est délimité, au niveau de ses deux ouvertures par des films 12 en une matière généralement souple et transparente, ce qui circonscrit une cavité où la sonication peut être effectuée.

Dans ce cas, la forme de la sonde peut être convexe de sorte que son application contre l'un des films 12, induit une déformation du film 12 concerné et augmente le couplage, synonyme d'amélioration de la lyse.

**RESULTATS EXPERIMENTAUX**

L'étude a porté sur deux types de sonotrodes, vibrant l'une à 20kHz, l'autre à 35kHz Ces sonotrodes ont été utilisées soit avec des tubes conventionnels (disponibles dans le commerce), soit avec des consommables de format carte.

Pour évaluer les différentes sonotrodes, deux modèles d'évaluation ont été choisis :

- les bactéries *(Staphylococcus epidermidis,* à titre de modèle), représentant le type de micro organimes le plus recherché dans des prélèvements à analyser.
- les levures *(Candida krusei,* à titre de modèle), réputées particulièrement difficiles à lyser, et également recherchées dans des prélèvements à analyser.

Deux types d'approches ont été choisies pour mener cette étude :

1 - Etude de différentes combinaisons de paramètres de sonication pour identifier les conditions optimales de lyse : l'analyse des lysats consiste à mesurer des densités optiques pour évaluer le pourcentage de lyse et à effectuer une migration sur gel d'agarose afin d'évaluer la libération des acides nucléiques ainsi que leur état de clivage. En ce qui concerne les électrophorèses sur gels d'agarose, aucune photographie n'est jointe du fait des difficultés de reproduction par photocopie, néanmoins les résultats obtenus sont commentés.

2 - Etude de sensibilité : détermination de la quantité minimale de micro-organismes lysés, dont les acides nucléiques peuvent être amplifiés et détectés, par toute technique d'amplification, notamment par PCR, avec le réglage optimal de paramètres déterminés lors de la première étape.

**III) SONICATION PAR SONOTRODES POUR TUBES A ESSAI**

**A - Sonotrode 20 kHz :**

Le dispositif utilisé pour cette étude est très bien décrit dans le chapitre I-A " Premie mode de réalisation " de l'appareil de lyse, ainsi que dans la figure 1. Ce dispositif comprend donc une sonotrode à 20 kHz, alimentée par un générateur, sur laquelle est positionné un tube de lyse, et un poids établissant le contact entre la sonotrode et le tube.

1°) Recherche du réglage optimal des paramètres :

Des levures *Candida krusei* ont été traitées par sonication directe avec le dispositif décrit ci-dessus, en faisant varier différents paramètres du protocole, et dans le but de maximiser leur lyse et la libération des acides nucléiques, et de minimiser le clivage de ces acides nucléiques.

*Conditions opératoires :*

Pour tous les essais, la puissance acoustique était comprise entre Ps = 0,8 x 25 = 20 W et Ps = 0,8 x 35 = 28 W. Dans les deux cas, le coefficient de conversion $\eta$ est de 80 %. On peut considérer que globalement l'énergie délivrée au tube était similaire dans tous les essais Cet ajustement de puissance permet simplement d'obtenir une agitation satisfaisante lorsque la quantité de billes est plus importante.

Les paramètres fixes sont les suivants :

- sonotrode à 20 kHz avec générateur Bioblock 300 W, poids de 1430 g et tube NUNC 1,8 ml,
- échantillon est une suspension de levures *Candida krusei* (souche n°9604058, collection bioMérieux), à environ $10^7$ levures/ml (D.O. à 550 nm de 0,600), dans un tampon à pH=8, contenant 80 mM d'HEPES, 1 mM d'EDTA et 2 % de lithium lauryl sulfate,
- volume échantillon = 600 $\mu$l, et
- billes de verre de diamètre 425 $\mu$m - 600 $\mu$m (acid-washed, Sigma).

Les paramètres variables sont indiqués dans le tableau n°1 :

- la puissance délivrée = Ps en Watt,
- le rapport cyclique en %,
- la durée de sonication en minutes, et
- la quantité de billes de verre en µl.

Les résultats sont évalués par :

- observation de l'agitation des billes : ne devant être ni trop faible (aucun mouvement), ni trop forte (accompagnée d'un échauffemment excessif de l'échantillon), et
- analyse de 20 µl de lysat par électrophorèse en gel d'agarose 0,8 %, et coloration au bromur d'éthidium (BET) ; en identifiant les bandes des acides nucléiques libérés sous forme intact (ADN génomique, rARN 18S et 26S) grâce à un marqueur de poids moléculaire.

*Résultats*

Tableau 1 :

| Sonication directe avec une sonotrode à 20 kHz, lyse de levures en tube et influence de la puissance acoustique, du rapport cyclique et de la quantité de billes de verre sur leur agitation, ainsi que sur la libération d'acides ucléiques de levures | | | | | | |
|---|---|---|---|---|---|---|
| Conditions n° | Ps (W) | Rapport cyclique (%) | Durée (mn) | Quantité billes (µl) | Résultats | |
| | | | | | Agitation | Acides nucl. |
| **1** | 24 | 20 | 5 | 30 | (+) | - |
| **2** | 24 | 20 | 5 | 60 | + | (+) |
| **3** | 23 | 50 | 5 | 30 | + | + |
| **4** | 20 | 50 | 5 | 60 | + | ++ |
| **5** | 21 | 80 | 5 | 60 | + | (+) |
| **6** | 28 | 80 | 2 | 90 | + | (+) |

Agitation des billes :        (+) faible, + correcte
Libération d'acides nucléiques intacts :    - absente, (+) faible, + visible, ++ visible, intense

Pour chaque condition expérimentale, deux essais ont été effectués.

Les résultats montrent que :

- l'agitation des billes de verre peut être parfaitement contrôlée en ajustant la puissance délivrée au tube (Ps),
- les levures ont pu être lysées et que les acides nucléiques ont pu être libérés sous forme non clivée: présence de signaux sur gel d'agarose, sous forme de bandes, correspondant à l'ADN génomique et aux ARN ribosomaux (notamment pour les conditions n° 3 et 4), et
- le réglage des paramètres a une incidence directe sur la quantité et le clivage des acides nucléiques libérés :

  * rapport cyclique : un réglage trop faible (20 %) ne libère pas assez de matériel un réglage trop fort (80 %) génère également des signaux faibles, dus à un clivage trop fort des acides nucléiques, et

\* quantité de billes : une quantité de billes plus importante (60 µl au lieu de 30 µl) en agitation correcte permet de libérer plus de matériel.

Les conditions retenues les plus intéressantes pour la suite des expériences sont donc celle décrite pour les essais n°4 de ce tableau n°1.

2°) Etude de sensibilité :

L'étude de sensibilité a été réalisée sur une gamme de dilutions de levures de $10^7$ à $10^2$ levures/essai. Ces levures ont été traitées par sonication directe dans les conditions optimales déterminées dans la première partie. En comparaison, la même gamme de dilutions a été traitée par une méthode de lyse de référence, le vortex, décrit dans la demande de brevet

*Conditions opératoires :*

Après préparation de suspensions de levures en eau à $10^2$, $10^3$, $10^4$ et $10^5$ levures/essai, celles-ci sont traitées par :

- sonication directe : conditions décrites précédemment dans le tableau n°1, conditions n°4,
- vortex : tube Falcon 5 ml, 600 µl d'échantillon, 150 µl de billes de verre de diamètre 425-600 µm, trois billes de verre de diamètre 2 mm, cinq billes de fer de diamètre 1,5 mm, vortex pendant 12min à puissance maximale.

Les lysats sont purifiés sur des colonnes Sephadex G-50 (Quick Spin columns for DNA purification, Boehringer Mannheim) selon le protocole du fournisseur, puis une amplification de 10 µl de lysat par PCR est réalisée, selon le protocole décrit par Makimura *et al* (*J. Med. Microbiol.*, 1994; 40: 358-364). 20µl de produit PCR sont ensuite analysés par éléctrophorèse sur gel d'agarose 1,2 % et coloration au bromure d'ethidium (BET).

*Résultats :*

Tableau 2 :

| Sonication directe avec une sonotrode à 20 kHz, lyse en tube - Sensibilité de détection de levures par PCR après selon deux traitements de lyse | | | | | | |
|---|---|---|---|---|---|---|
| levures détectées par essai | $10^7$ | $10^6$ | $10^5$ | $10^4$ | $10^3$ | $10^2$ |
| lyse par sonication directe | ++ | ++ | ++ | + | - | - |
| lyse par vortex | ++ | ++ | ++ | + | - | - |

Les deux types de lyses sont les suivantes :

- lyse par sonication directe avec une sonotrode de 20 kHz, et
- lyse par vortex.

Signal sur gel : - non visible, + visible, ++ visible et intense

Pour chaque condition expérimentale, deux essais ont été effectués.

Les résultats montrent que :

- les lysats obtenus par sonication directe dans les conditions optimales sont bien amplifiables par PCR : présence de bandes visibles et intenses correspondant bien à la taille du produit PCR recherché, et
- après lyse par sonication directe ainsi que par vortex, la sensibilité de détection par cette méthode de PCR est de

$10^4$ levures/essai. La lyse par sonication permet donc d'obtenir les mêmes performances qu'une méthode de lyse de référence.

3°) <u>Conclusion :</u>

Ces essais ont permis de démontrer la faisabilité de la lyse par sonication directe à 20 kHz. Ils ont également montré qu'il est possible d'ajuster les paramètres de sonication afin de maximiser la libération d'acides nucléiques de levures et de minimiser leur clivage. Enfin, ces paramètres permettent d'obtenir des acides nucléiques amplifiables par PCR et d'atteindre une sensibilité de détection équivalente à celle d'une méthode de lyse de référence.

**B - Sonotrode 35 kHz :**

Le dipositif de sonication utilisé est représenté dans la figure 2, et est très bien décrit dans le chapitre I-B " Deuxième mode de réalisation " de l'appareil de lyse. Les avantages d'une sonication à 35 kHz au lieu de 20 kHz sont expliqués dans ce même chapitre. Par ailleurs, ce dispositif de lyse présente une plus grande surface de contact entre tube et sonotrode ; les avantages de ceci sont expliqués dans le chapitre II-A " Premier mode de réalisation " du récipient contenant l'échantillon à traiter.

1°) <u>Recherche du réglage optimal des paramètres :</u>

Le but a été d'identifier les paramètres de sonication permettant de maximiser la lyse de bactéries et de levures, et donc de maximiser la libération des acides nucléiques en contrôlant parfaitement leur clivage.

*Conditions opératoires :*

Pour les essais suivants, la puissance acoustique est définie par l'amplitude vibratoire des ultrasons générés par la sonotrode. Cette amplitude est donnée en pourcentage de la puissance du générateur fournissant l'énérgie électrique au sonicateur.

<u>Les paramètres fixes sont les suivants :</u>

- sonotrode à 35 kHz avec générateur SODEVA 500 W, poids de 1430 g et tube NUNC 1,8 ml,
- échantillon :

  * pour les essais sur les levures : suspension de levures *Candida krusei* (souche n°9604058, collection bioMérieux) à environ $2x10^7$ levures/ml (D.O. à 550 nm de 0,950), dans un tampon à pH=8,5 contenant 80 mM d'HEPES, 1 mM d'EDTA et 2 % de lithium lauryl sulfate, et
  * pour les essais sur les bactéries : suspension de bactéries *Staphylococcus epidermidis* (souche n°A054, collection bioMérieux), à environ $10^9$ bactéries/ml (D.O. à 550 nm de 0,900) dans un tampon à pH=7,2 contenant 30 mM de Tris, 5 mM d'EDTA et 100 mM de NaCl,

- volume échantillon = 600 µl, et
- billes de verre :

  * pour les essais sur les levures : 90 µl de billes de diamètre 425 µm - 600 µm (acid-washed, Sigma), et
  * pour les essais sur les bactéries : 120 µl de billes de diamètre 106 µm (VIA I).

<u>Les paramètres variables sont indiqués dans les tableau n°2 et n°3:</u>

- l'amplitude vibratoire en %,
- la puissance délivrée = Ps en Watt, étant directement liée à l'amplitude,
- la durée des pulses de sonication (Ton) et la durée des repos (Toff) en minutes, ces durées définissent le rapport cyclique: Ton / (Ton + Toff), et
- la durée totale de sonication en minutes.

Les résultats sont évalués par :

- observation de l'agitation des billes,
- mesure de la densité optique à 550 nm des suspensions cellulaires avant les traitements de lyse, ainsi qu'après lyse. Le pourcentage de lyse approximatif est déterminé par :

$$(D.O._{550nm} \text{ après lyse}) / ((D.O._{550nm} \text{ avant lyse}) \times 100),$$

et

- analyse de 20 µl de lysat par électrophorèse en gel d'agarose 0,8 %, et coloration au bromure d'éthidium (BET) ; en identifiant les bandes des acides nucléiques libérés sous forme intacte (ADN génomique, rARN 18S et 26S) grâce à un marqueur de poids moléculaire. Les acides nucléiques clivés apparaissent situé en-dessous de ces bandes.

*Résultats : Essais sur levures*

| Conditions | Ampl. | Ps | Ton | Toff | Durée | Résultats | | | |
|---|---|---|---|---|---|---|---|---|---|
| | | | | | | lyse | acides nucléiques | | |
| n° | (%) | (W) | (sec) | (sec) | (min) | (%) | ADN | rARN | clivés |
| 1 | 50 | 15 | 10 | 10 | 10 | 15% | ++ | ++ | + |
| 2 | 50 | 15 | 10 | 10 | 15 | 19% | ++ | +++ | + |
| 3 | 50 | 15 | 10 | 2 | 10 | 27% | ++ | + | ++ |
| 4 | 80 | 20 | 10 | 10 | 10 | 26% | ++ | + | ++ |

**<u>Tableau 3 : Sonication directe avec une sonotrode à 35 kHz, lyse en tube - Influence de la puissance acoustique, du rapport cyclique et de la durée de sonication sur le pourcentage de lyse de levures, ainsi que sur la libération des acides nucléiques et leur état de clivage</u>**

Libération des acides nucléiques :     - absente, + visible, ++ visible et intense, +++ visible et très intense.

Pour chaque condition expérimentale, deux essais ont été effectués.

*Résultats :*    *Essais sur bactéries*

| Conditions | Ampl. | Ps | Ton | Toff | Durée | Résultats | | | |
| | | | | | | lyse | acides nucléiques | | |
| n° | (%) | (W) | (sec) | (sec) | (min) | (%) | ADN | rARN | clivés |
|---|---|---|---|---|---|---|---|---|---|
| 1 | 50 | 15 | 10 | 10 | 10 | 30% | ++ | ++ | ++ |
| 2 | 50 | 15 | 10 | 10 | 15 | 49% | ++ | ++ | ++ |
| 3 | 50 | 15 | 10 | 2 | 10 | 40% | ++ | ++ | ++ |
| 4 | 80 | 20 | 10 | 10 | 10 | 53% | + | + | ++ |
| 5 | 80 | 20 | 10 | 10 | 15 | 59% | - | - | +++ |

**<u>Tableau 4 : Sonication directe avec une sonotrode à 35 kHz, lyse en tube - Influence de la puissance acoustique, du rapport cyclique et de la durée de sonication sur le pourcentage de lyse de bactéries, ainsi que sur la libération des acides nucléiques et leur état de clivage</u>**

Libération des acides nucléiques:    - absente, + visible, ++ visible et intense, +++ visible et très intense

Pour chaque condition expérimentale, deux essais ont été effectués.

*Résultats concernant l'agitation des billes :*

    **Puissance 15 W (amplitude 50 %) :** agitation homogène des billes dans la moitié inférieure du volume du liquide, en formant un dôme d'agitation au centre. Les billes subissent de petits mouvements, en se frottant entre elles.
    **Puissance 20 W (amplitude 80 %) :** agitation plus violente; les billes réalisent des mouvements plus amples, occupant un volume plus important du liquide. L'échauffement du liquide est plus important également.

Les résultats concernant l'agitation montrent que :

- une puissance de 15 W suffit pour générer une agitation correcte des billes de verre,
- par rapport à la sonotrode de 20 kHz, celle de 35 kHz nécessite une puissance moindre pour générer une agitation correcte des billes de verre (15 W au lieu de 20 W), et ceci pour une quantité plus importante de billes (90 μl et 120 μl au lieu de 60 μl), et
- grâce à une transmission des ultrasons par une surface plus importante (toute la base du tube), l'agitation des billes est plus homogène et l'échauffement du liquide est moindre.

Les résultats concernant la lyse montrent que :

- les bactéries et les levures ont pu être lysées et leurs acides nucléiques ont pu être libérés,
- le réglage des paramètres a une incidence directe sur la quantité et le clivage des acides nucléiques libérés :

| Puissance : | - une amplitude vibratoire élevée (80 %) permet d'augmenter |
| (amplitude) | légèrement le pourcentage de lyse et la quantité d'acides nucléiques libérée, et elle provoque un clivage intense. |
| | - une amplitude de 50 % libère une quantité satisfaisante d'acides nucléiques en préservant totalement l'ADN génomique et partiellement les ARN ribosomaux, |
| Rapport cyclique : | un rapport cyclique élevé (Ton 10sec/ Toff 10sec) a le même effet qu'une amplitude élevée, |
| Durée de sonication : | une durée importante (15 mn) permet d'augmenter le pourcentage de lyse et la quantité d'acides nucléiques libérée sans influer sur leur état de clivage, |

- la condition retenue pour obtenir un maximum d'acides nucléiques sous forme non-clivée est une sonication longue à amplitude et rapport cyclique faibles (conditions n°2, tableaux n°2 et n°3), et
- la condition retenue pour obtenir un maximum d'acides nucléiques sous forme clivée est une sonication longue à amplitude élevée (condition n°5, tableau n°2).

2°) <u>Etude de sensibilité :</u>

L'étude de sensibilité a été réalisée sur une gamme de dilutions de levures de $10^6$ à $10^2$ levures/essai. Ces levures ont été traitées par sonication directe dans différentes conditions évaluées dans la première partie :

1. la condition retenue pour obtenir un maximum d'acides nucléiques sous forme non-clivée,
2. cette même condition, en augmentant simplement le rapport cyclique,
3. toujours la condition n°1, mais en augmentant simplement l'amplitude.

Dans la première partie, il a été montré que ces deux dernières conditions conduisent à un pourcentage de lyse plus important, mais aussi à un clivage plus fort des acides nucléiques. Par ailleurs, la même gamme de dilutions a été traitée par une méthode de lyse de référence, le vortex, décrit dans la demande de brevet WO9915621.

*Conditions opératoires :*

Après préparation de suspensions de levures en eau à $10^2$, $10^3$, $10^4$, $10^5$, $10^6$ et $10^7$ levures/essai, celles-ci sont traitées par :

- sonication directe : conditions n°1, 2 et 3 décrites dans la première partie, tableaux n°2 et 3,
- vortex : tube Falcon 5 ml, 600 µl d'échantillon, 150 µl de billes de verre de diamètre 425-600 µm, trois billes de verre de diamètre 2 mm, cinq billes de fer de diamètre 1,5 mm, vortex pendant 12 mn à puissance maximale.

Les lysats sont purifiés sur des colonnes Sephadex G-50 (Quick Spin columns for DNA purification, Boehringer Mannheim) selon le protocole du fournisseur, puis une amplification de 10 µl de lysat par PCR est réalisée, selon le protocole décrit par Makimura *et al* (*J. Med. Microbiol.,* 1994; 40: 358-364). 20 µl de produit PCR sont ensuite analysés par éléctrophorèse sur gel d'agarose 1,2 % et coloration au bromure d'ethidium (BET).

*Résultats :*

Tableau 5 :

| Sonication directe avec une sonotrode à 20 kHz - Sensibilité de détection de levures par PCR après différents traitements de lyse | | | | | |
|---|---|---|---|---|---|
| levures détectées par essai | $10^6$ | $10^5$ | $10^4$ | $10^3$ | $10^2$ |
| lyse par sonication directe n°1 | ++ | ++ | + | - | - |
| lyse par sonication directe n°2 | ++ | ++ | (+) | - | - |
| lyse par sonication directe n°3 | ++ | ++ | (+) | - | - |
| lyse par vortex | ++ | ++ | + | (+) | - |

Ces différents traitements de lyse sont :

- lyse par sonication directe avec une sonotrode à 20 kHz dans trois conditions, et
- lyse par vortex.

Signal sur gel :   - non visible, (+) faible, + visible, ++ visible et intense

Pour chaque condition expérimentale, deux essais ont été effectués.

Les résultats montrent que :

- les lysats obtenus par sonication directe dans les conditions optimales sont bien amplifiables par PCR ; il y a présence de bandes visibles et intenses correspondant bien à la taille du produit PCR recherché,
- après lyse par sonication directe dans trois conditions différentes, jusqu'à $10^4$ levures par essai sont détectées par PCR ; néanmoins, les signaux observés pour $10^4$ levures par essai sont plus faibles, lorsque la sonication est effectuée dans des conditions clivant plus fortement les acides nucléiques (conditions n°2 et 3), et
- la sensibilité de détection obtenue après lyse par sonication directe est très proche de celle obtenue après lyse par une méthode de référence, le vortex.

3°) Conclusion :

Ces essais ont démontré la faisabilité de la lyse par sonication directe à 35 kHz, sur deux organismes différents (bactéries et levures). Il a également été démontré qu'un réglage judicieux de quelques paramètres de sonication importants permet de maximiser la lyse de ces organismes, tout en contrôlant parfaitement l'état de clivage des acides nucléiques libérés. Ces paramètres importants de sonication ont été identifiés et les réglages optimaux ont été déterminés. Par ailleurs, il a été montré que la lyse par sonication directe à 35 kHz permet de libérer des acides nucléiques amplifiables par PCR, et que les conditions de sonication identifiées comme favorables à une amplification par PCR permettent d'obtenir une sensibilité de détection très proche de celle obtenue avec une méthode de lyse de référence, le vortex. Les conditions de sonication clivant plus fortement les acides nucléiques sont légèrement moins favorables à une amplification par PCR. Néanmoins, un fort clivage des acides nucléiques peut être très favorable à certaines autres applications en biologie moléculaire, comme p. ex. pour une purification des acides nucléiques utilisant des processus d'hybridation lesquelles sont favorisés par une fragmentation préalable des acides nucléiques.

**C - Sonotrode 35kHz multi-tubes :**

Le concept d'une sonication directe simultanée de plusieurs tubes est représenté dans la figure 3, et est décrit dans le chapitre I-C " Troisième mode de réalisation " de l'appareil de lyse. Les essais suivants ont été réalisés avec un dispositif qui permet la sonication de douze tubes simultanément, et qui présente par ailleurs les mêmes caractéristiques que la sonotrode 35 kHz utilisée pour les essais de la partie I-B (même fréquence de sonication, même surface de contact tube-sonotrode, etc.). La particularité de ce dipositif de sonication multi-tubes réside dans la possibilité d'ajuster la pression exercée sur chacun des douze tubes afin d'obtenir une bonne reproductibilité des contacts tube-sonotrode, et donc de l'efficacité de lyse d'un tube à l'autre.

1°) Ajustement du couplage tubes - sonotrode pour une sonication reproductible :

La pression exercée par le poids sur chacun des douze tubes a été mesurée, puis ajustée pour obtenir une pression homogène pour tous les tubes. Cette homogénéité a été confirmée par un comportement homogène des billes lors de la sonication dans les conditions optimales déterminées dans la partie I-B.

*Conditions opératoires :*

La force appliquée sur chaque tube a été mesurée à l'aide d'un capteur de force (référence BC302 3KG MV/V, fournisseur C2AI, France) en forme disque, d'un diamètre = 16 mm, et d'une épaisseur = 4,5 mm, positionné sur un tube tronqué, afin que la hauteur du tube de test associée à l'épaisseur du capteur de force soient identiques à la hauteur des tubes échantillons. Le capteur est relié à un indicateur digital (type 48 x 96 programmable, fournisseur C2AI, France) afin de visualiser une tension représentative de la force de compression.

Pour les mesures, onze tubes échantillons et le tube test avec capteur de force ont été positionnés sur les douze positions de la sonotrode. Le tube de test, muni du capteur, est successivement déplacé de la position 1 vers la position 2 puis vers la position 3... jusqu'à la position 12, afin de relever la force de compression appliquée sur les tubes à chaque position. Trois séries de mesure ont été réalisées. L'écart type de répartition de la force de couplage calculé sur la moyenne des trois séries de mesures montre de fortes disparités avant réglage puisque que le coefficient de variation (CV) est de l'ordre de 10% (voir tableau 6).

Le réglage de la force d'appui est ensuite effectué pour chaque position en présence du capteur par réglage du ressort de compression de chaque axe d'appui.

Après réglage, on constate que le CV des valeurs de force de compression a été ramené à une valeur inférieur à 1 %.

*Résultats*

Tableau 6 :

| Sonication directe avec une sonotrode à 35 kHz pour multi-tubes - Pression exercée sur les différents tubes | | | | | |
|---|---|---|---|---|---|
| Position | Effort Série 1 | Effort Série 2 | Effort Série 3 | Moyenne | *Après Réglage (24/12)* |
| 1 | 0,625 | 0,595 | 0,605 | 0,608 | *0,573* |
| 2 | 0,575 | 0,567 | 0,572 | 0,571 | *0,577* |
| 3 | 0,595 | 0,595 | 0,604 | 0,598 | *0,574* |
| 4 | 0,606 | 0,609 | 0,592 | 0,602 | *0,563* |
| 5 | 0,684 | 0,694 | 0,684 | 0,687 | *0,572* |
| 6 | 0,651 | 0,659 | 0,659 | 0,656 | *0,573* |
| 7 | 0,5 | 0,508 | 0,527 | 0,512 | *0,572* |
| 8 | 0,502 | 0,519 | 0,513 | 0,511 | *0,579* |
| 9 | 0,484 | 0,494 | 0,494 | 0,491 | *0,571* |
| 10 | 0,552 | 0,523 | 0,518 | 0,531 | *0,571* |
| 11 | 0,582 | 0,569 | 0,571 | 0,574 | *0,572* |
| 12 | 0,526 | 0,556 | 0,548 | 0,543 | *0,572* |
| **Moyenne** | 0,574 | 0,574 | 0,574 | 0,574 | 0,572 |
| **Ecart type %** | 11,0 | 10,6 | 10,2 | 10,5 | 0,7 |

*Caractérisation du mouvement de lyse :*

Après homogénéisation des pressions exercées sur les douze tubes, le caractère plus ou moins homogène du mouvement des billes lors de la sonication a été évalué visuellement. La sonication a été réalisée exactement comme décrit dans la partie III - B, en appliquant la condition n°2 décrite dans les tableaux n°3 et 4 (déterminée comme optimale pour une lyse maximale et une préservation maximale des acides nucléiques), et en utilisant de l'eau comme " échantillon ". Par ailleurs, le poids agissant sur les douze tubes a été augmenté graduellement (2,7 kg, 3,7 kg, 4,6 kg, 5,6 kg), ce qui augmente le contact entre tubes et sonotrode.

Il a été constaté qu'un poids trop faible (2,7kg) conduit à une très grande variabilité du mouvement des billes d'un tube à l'autre. En effet, ce poids ne suffit pas à établir un contact suffisant entre les douze tubes et la sonotrode, puisque les billes dans certains tubes sont au repos.

A partir d'un poids seuil par contre (3,7kg), le mouvement des billes présente le même caractère dans chacun des douze tubes : agitation homogène des billes dans la moitié inférieure du volume du liquide, en formant un dôme d'agitation au centre. Les billes subissent de petits mouvements, en se frottant entre elles.

2°) Conclusion :

Il a été montré qu'il est possible de transposer le principe de sonication directe à 35 kHz avec une surface de contact tube-sonotrode optimale (décrit dans la partie I-B) à un traitement simultané de plusieurs tubes. Par ailleurs, il est possible d'obtenir un mouvement de lyse homogène dans tous les tubes en ajustant les forces de contact tubes - sonotrode. Ces deux observations démontrent la possibilité d'utiliser le principe de la sonication directe dans un automate pour traiter un certain nombre d'échantillons de façon reproductible.

## IV) SONICATION PAR SONOTRODES POUR CONSOMMABLES DE FORMAT CARTE

### Sonotrode à 20kHz pour cartes :

Le dispositif de sonication en carte est représenté dans les figures 4 à 6. Ces figures montrent également deux possibilités de positionnement ainsi que le comportement du liquide et des billes pendant la sonication. La sonotrode utilisée pour ce dispositif est celle décrite dans le chapitre I-A " Premier mode de réalisation " de l'appareil de lyse, mais en adaptant un embout plat de diamètre 13 mm. Le consommable de format carte est décrit dans le chapitre II-B " Deuxième mode de réalisation " du récipient contenant l'échantillon à traiter. Ce consommable est en polystyrène (d'autres plastiques compatibles avec les analyses biologiques peuvent être utilisés), a les dimensions 40 x 40 x 4 mm et dispose d'une ouverture circulaire au centre, appelée puits, de diamètre variable, pour accueillir l'échantillon et les billes de verre. Le fond de ce puits est constitué soit de plastique de 0,3 mm d'épaisseur, soit d'un film autocollant polypropylène. Ce film sert également à fermer le consommable.

1°) Evaluation des paramètres liés au format carte :

Seuls les paramètres liés à l'utilisation de cartes ont été évalués, en choisissant des valeurs fixes pour les autres paramètres de sonication, déjà étudiés dans la partie I-A. Les essais de lyse ont été réalisés avec des levures et des bactéries, et l'efficacité de lyse ainsi que l'état de clivage des acides nucléiques libérés ont été déterminés.

*Conditions opératoires :*

Les paramètres fixes sont les suivants :

- sonotrode à 20 kHz avec générateur Bioblock 300 W et consommable décrits ci-dessus, la carte étant en position horizontale,
- puissance :

  • pour les essais sur les levures : 25 W, et
  • pour les essais sur les bactéries : 13-14 W,

- rapport cyclique de 80 % et durée de sonication 5 mn,
- échantillon :

  • pour les essais sur les levures : suspension de levures *Candida krusei* (souche n°9604058, collection bioMérieux) à environ $2 \times 10^7$ levures/ml (D.O. à 550 nm de 0,950), dans un tampon à pH=8,5 contenant 80 mM d'HEPES, 1 mM d'EDTA et 2 % de lithium lauryl sulfate, et
  • pour les essais sur les bactéries : suspension de bactéries *Staphylococcus epidermidis* (souche n°A054, collection bioMérieux), à environ $8,5 \times 10^8$ bactéries/ml (D.O. à 550 nm de 0,720), dans le même tampon, et

- billes de verre :

  • pour les essais sur les levures : billes de diamètre 425 μm - 600 μm (acid-washed, Sigma), et
  • pour les essais sur les bactéries : billes de diamètre 106 μm (VIA I).

Les paramètres variables sont indiqués dans les tableaux n°6 et n°7 :

- le diamètre du puits en mm,
- la nature du fond du puits (film transparent souple ou fond en plastique),

- le volume de l'échantillon en µl, et
- le volume de billes de verre en µl.

Les résultats sont évalués par :

- observation de l'agitation des billes, et
- analyse de 20 µl de lysat par électrophorèse en gel d'agarose 0,8 %, et coloration au bromure d'éthidium (BET) ; en identifiant les bandes des acides nucléiques libérés sous forme intacte (ADN génomique, rARN 18S et 26S) grâce à un marqueur de poids moléculaire.

Les acides nucléiques clivés apparaissent au-dessous de ces bandes.

*Résultats : Essais sur bactéries*

| Conditions n° | Paramètres " carte " | | | | Résultats | | |
|---|---|---|---|---|---|---|---|
| | diamètre (mm) | fond | volume (µl) échantillon | volume (µl) billes | acides nucléiques | | |
| | | | | | ADN | rARN | clivés |
| 1 | 15 | film | 200 | 60 | + | ++ | - |
| 2 | 15 | plast. | 200 | 60 | - | + | + |
| 3 | 20 | film | 300 | 90 | + | ++ | - |
| 4 | 20 | plast. | 300 | 90 | - | + | + |
| 5 | 25 | film | 600 | 180 | + | + | - |
| 6 | 25 | plast. | 600 | 180 | + | + | + |
| 7 | 30 | film | 900 | 180 | (+) | (+) | - |
| 8 | 30 | plast. | 900 | 180 | + | (+) | - |

**Tableau 7 : Sonication directe avec une sonotrode à 20 kHz, lyse en carte - Influence des paramètres liés au format 'carte' (taille et fond du puits, volume de l'échantillon, quantité de billes) sur la lyse de bactéries ; et notamment la quantité d'acides nucléiques libérés et leur état de clivage.**

Libération des acides nucléiques : - absente, + visible, ++ visible et intense
Remarque : pour la condition n°8, la carte est en position verticale.

*Résultats : Essais sur levures*

| | Paramètres " carte " | | | | Résultats | | |
|---|---|---|---|---|---|---|---|
| Conditions | diamètre | fond | volume (µl) | volume (µl) | acides nucléiques | | |
| n° | (mm) | | échantillon | billes | ADN | rARN | clivés |
| 1 | 15 | film | 600 | 30 | + | + | - |
| 2 | 15 | plast. | 300 | 30 | (+) | (+) | (+) |
| 3 | 30 | film | 600 | 180 | + | ++ | - |
| 4 | 30 | plast. | 600 | 180 | - | (+) | (+) |

**Tableau 8 : Sonication directe avec une sonotrode à 20kHz, lyse en carte - Influence des paramètres liés au format " carte " (taille et fond du puits, volume de l'échantillon, quantité de billes) sur la lyse de levures ; et notamment la quantité d'acides nucléiques libérés et leur état de clivage.**

Libération des acides nucléiques :     - absente, + visible, ++ visible et intense

Remarque : pour les conditions n° 3 et 4, la durée de sonication est de 10 mn.

*Résultats concernant l'agitation :*

**Position horizontale, avec fond en film souple :** Les ultrasons générés par la sonotrode éjectent le liquide et les billes vers la périphérie du puits (voir figure 6). Les billes subissent de petits mouvements d'agitation très rapide, en se frottant entre elles. Cette agitation est principalement due aux ultrasons qui sont transmis latéralement par le film. Il n'y a pas dégradation du film, ni d'échauffement notable du liquide.
**Position horizontale, avec fond en plastique :** Le liquide et les billes sont localisés de la même façon que ci-dessus, mais l'agitation des billes est clairement plus faible. Par contre, il y a échauffement intense du fond en plastique, et donc du liquide.
**Position verticale, avec fond en film souple :** Le liquide et les billes sont localisés dans le bas du puits, juste en face de la sonotrode, et donc dans un volume plus petit qu'en position horizontale. L'agitation des billes est assez forte, mais il n'y a pas d'échauffement notable du liquide.

Les résultats montrent que :

- les bactéries et les levures ont pu être lysées et leurs acides nucléiques ont pu être libérés,
- la sonication en position horizontale et avec un fond de puits en film souple permet une lyse très efficace et la libération d'une quantité importante d'acides nucléiques (signaux très intenses sur gel), sans aucun clivage ni de l'ADN ni des ARN ribosomaux. En effet, le film très fin, résistant et souple permet un très bon couplage entre la sonotrode et le film, donc une agitation forte des billes, et une transmission des ultrasons avec relativement peu de dissipation par effet Joule, donc peu d'échauffement. De plus, le confinement du liquide à la périphérie du puits, hors de l'axe acoustique, réduit notablement le phénomène de cavitation dans le liquide,

- la sonication avec un fond en plastique libère une quantité plus faible d'acides nucléiques, tout en provoquant un clivage plus fort des acides nucléiques (ADN et rARN). Ceci est clairement dû à l'agitation plus faible des billes et à l'échauffement important du fond de la carte et du liquide. Ces deux phénomènes s'expliquent par un mauvais couplage entre la sonotrode et le fond rigide de la carte, ainsi qu'à un effet Joule plus important, et

- plus le diamètre du puits de lyse augmente, plus l'efficacité de lyse est réduite et moins d'acides nucléiques sont libérés. L'éloignement du liquide et des billes de la sonotrode au centre est donc à éviter puisque l'effet des ultrasons est réduit. Il est donc recommandé d'utiliser des cartes avec un puits de diamètre proche de celui de la sonotrode.

2°) <u>Conclusion :</u>

La faisabilité d'une sonication directe en consommable de format carte a clairement été démontrée. Ces essais ont également permis d'identifier des conditions d'utilisation de cartes conduisant à une grande efficacité de lyse avec une totale préservation des acides nucléiques libérés (puits de diamètre proche de celui de la sonotrode, et couplage par un film fin et souple). Cette préservation des acides nucléiques est très avantageuse pour des analyses de biologie moléculaire nécessitant un état parfaitement intact des acides nucléiques. Par ailleurs, l'utilisation de ce type de consommable pour la sonication directe est très intéressante, car elle permettrait d'intégrer d'éventuels traitements et analyses ultérieurs de l'échantillon dans la même carte. De cette façon, la lyse de micro-organismes et leur analyse et identification pourraient être réalisés dans un seul consommable ce qui réduirait les risques de contamination inter-échantillon et permettrait une automatisation aisée du procédé.

**REFERENCES**

1. Appareil
2. Sonotrode ou sonde
3. Générateur
4. Tube ou récipient
5. Echantillon contenu dans le tube 4 ou la carte 10
6. Moyen de mise sous pression ou plateau supérieur
7. Poids de lestage
8. Surface active de la sonotrode 2
9. Booster
10. Carte ou récipient
11. Billes de verre
12. Films
13. Puits
14. Embout plat
15. Sonotrode ou sonde
16. Surface active de la sonotrode 15
17. Transducteur
18. Base
19. Plaque support
20. Plaque de pression
21. Elément de compression associé à un tube 4
F1. Déplacement de l'échantillon 5
F2. Vibrations du film 12

**Revendications**

**1.** Appareil (1), comprenant une sonotrode (2) destinée à générer des ultrasons de plus ou moins forte puissance dans au moins un échantillon biologique (5) contenant des cellules à lyser, échantillon (5) qui est contenu dans au moins un récipient (4) adapté à cette usage, la sonotrode (2) est directement en contact avec le ou les récipients (4), contenant l'échantillon à lyser (5), en l'absence de fluide entre lesdites surfaces en contact entre la sonotrode (2) et le récipient (4), **caractérisé par le fait que** la sonotrode (2) possède une surface active (8) qui épouse la forme de la surface extérieure de tout ou partie de chaque récipient (4) contenant l'échantillon à lyser ; la surface active (8) de la sonotrode (2) en contact avec le récipient (4) est de forme concave.

2. Appareil, comprenant une sonotrode (15) destinée à générer des ultrasons de plus ou moins forte puissance dans au moins un échantillon biologique (5) contenant des cellules à lyser, échantillon (5) qui est contenu dans au moins un récipient (10) adapté à cette usage, la sonotrode (15) est directement en contact avec le ou les récipients (10), contenant l'échantillon à lyser (5), en l'absence de fluide entre lesdites surfaces en contact entre la sonotrode (15) et le récipient (10), **caractérisé par le fait que** la sonotrode (15) possède une surface active (16) qui épouse la forme de la surface extérieure de tout ou partie de chaque récipient (10) contenant l'échantillon à lyser ; la surface active (16) de la sonotrode (15) en contact avec la surface extérieure du récipient (10) est de forme convexe.

3. Appareil, selon l'une quelconque des revendications 1 ou 2, **caractérisé par le fait que** la surface du récipient (10), qui coopère avec la sonotrode (15), est flexible de sorte qu'elle vient épouser la surface active (16) de ladite sonotrode (15).

4. Appareil, selon l'une quelconque des revendications 1 à 3, **caractérisé par le fait que** la sonotrode (2 ou 15) coopère avec au moins une bille de verre (11) située dans l'échantillon (5) contenu dans un récipient (4 ou 10).

5. Appareil, selon l'une quelconque des revendications 1 à 4, **caractérisé par le fait que** chaque bille (11) a un diamètre de 90 à 150 et préférentiellement de 100 micromètres (μm) dans le cas d'une lyse de bactéries, et un diamètre de 150 à 1500 et préférentiellement de 500 μm dans le cas d'une lyse de levures.

6. Appareil, selon l'une quelconque des revendications 1 à 5, **caractérisé par le fait qu'**il comporte au moins deux sonotrodes (2 ou 15).

7. Appareil, selon l'une quelconque des revendications 1 à 6, **caractérisé par le fait que** chaque récipient (4 ou 10) est en contact physique avec la ou les sonotrodes (2 ou 15) par un moyen de mise sous pression (6).

8. Appareil, selon l'une quelconque des revendications 1 à 7, **caractérisé par le fait que** chaque sonotrode (2 ou 15) possède une fréquence d'émission des ultrasons comprise entre 20 et 50 kHz, plus précisément entre 30 et 40 kHz et préférentiellement de sensiblement 35 kHz.

9. Procédé de lyse par ultrasons de cellules contenues dans un échantillon biologique (5) présent dans un récipient (4 ou 10), qui utilise au moins une sonotrode (2 ou 15), **caractérisé en ce qu'**il consiste à :

   - positionner le récipient (4 ou 10) au contact direct avec la surface active concave ou convexe de la ou des sonotrodes (2 ou 15), et
   - activer ladite ou lesdites sonotrodes (2 ou 15) pendant un temps suffisant pour lyser les cellules contenues dans l'échantillon (5) en conservant les ADN et/ou ARN libérés pour permettre leur utilisation ultérieure, par exemple dans une étape d'amplification.

10. Procédé de lyse par ultrasons de cellules contenues dans un échantillon biologique (5) présent dans un récipient (4 ou 10), qui utilise au moins une sonotrode (2 ou 15), **caractérisé en ce qu'**il consiste à :

   - positionner le récipient (4 ou 10) au contact direct avec la surface active concave ou convexe de la ou des sonotrodes (2 ou 15), et
   - activer ladite ou lesdites sonotrodes (2 ou 15) pendant un temps suffisant pour lyser les cellules contenues dans l'échantillon (5) en clivant les ADN et/ou ARN libérés, tout en permettant leur utilisation ultérieure, par exemple dans une étape d'amplification.

11. Procédé, selon l'une quelconque des revendications 9 ou 10, **caractérisé en ce que**, préalablement à l'activation de la ou des sonotrodes (2 ou 15), le récipient (4 ou 10) est pressé à l'encontre de la surface active (8 ou 16) de ladite ou desdites sonotrodes (2 ou 15).

**12.** Procédé, selon l'une quelconque des revendications 9 à 11, **caractérisé en ce que** l'activation de chaque sonotrode (2 ou 15) s'effectue dans les conditions suivantes :

- durée de la sonication 10 à 15 minutes,
- rapport cyclique compris entre 40 et 60 % et préférentiellement 50 %, et
- puissance 10 à 30 W.

**13.** Procédé, selon la revendication 12, **caractérisé en ce que** l'activation de chaque sonotrode (2 ou 15) consiste en une série d'impulsions dont la durée est comprise entre 5 et 20 secondes et préférentiellement entre 10 et 15 secondes

**Fig. 1**

4

5

2

9

3

17

**Fig. 2**

**Fig. 3**

**Fig. 4**

**Fig. 5**

**Fig. 6**

<table>
<tr><td colspan="3"><strong>Office européen</strong><br><strong>des brevets</strong></td><td colspan="2"><strong>RAPPORT DE RECHERCHE EUROPEENNE</strong></td><td>Numéro de la demande<br>EP 04 01 2952</td></tr>
</table>

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 337 690 A (GENE TRAK SYSTEMS) 18 octobre 1989 (1989-10-18) * page 3, ligne 30 - ligne 52; revendications; figure * | 1,3-5, 8-11 | C12M1/33 C12M3/08 |
| A | EP 0 271 448 A (BATTELLE MEMORIAL INSTITUTE) 15 juin 1988 (1988-06-15) * revendications; figures * | 1,3-5 | |
| A | GB 938 163 A (BOOTS PURE DRUG CIE LTD) 2 octobre 1963 (1963-10-02) * revendications; exemples * | 4,5 | |
| A | EP 0 769 475 A (YOSHIDA KANJI ;REMODELING 21 CO LTD (JP)) 23 avril 1997 (1997-04-23) * revendications 1,3,9; figure 4 * | | |
| A | EP 0 604 742 A (MALMROS HOLDING INC) 6 juillet 1994 (1994-07-06) | | |

**DOMAINES TECHNIQUES RECHERCHES (Int.Cl.7)**

C12M
C12N

Le présent rapport a été établi pour toutes les revendications

| Lieu de la recherche | Date d'achèvement de la recherche | Examinateur |
|---|---|---|
| La Haye | 12 août 2004 | Van der Schaal, C |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**          EP 04 01 2952

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

12–08–2004

| Document brevet cité au rapport de recherche | | Date de publication | Membre(s) de la famille de brevet(s) | | Date de publication |
|---|---|---|---|---|---|
| EP  0337690 | A | 18–10–1989 | US | 4983523 A | 08–01–1991 |
| | | | AT | 91155 T | 15–07–1993 |
| | | | DE | 68907370 D1 | 05–08–1993 |
| | | | DE | 68907370 T2 | 04–11–1993 |
| | | | EP | 0337690 A1 | 18–10–1989 |
| | | | JP | 2043000 A | 13–02–1990 |
| EP  0271448 | A | 15–06–1988 | CH | 667599 A5 | 31–10–1988 |
| | | | DK | 648587 A | 12–06–1988 |
| | | | EP | 0271448 A2 | 15–06–1988 |
| | | | JP | 1027649 A | 30–01–1989 |
| GB  938163 | A | 02–10–1963 | AUCUN | | |
| EP  0769475 | A | 23–04–1997 | JP | 9108676 A | 28–04–1997 |
| | | | AT | 190043 T | 15–03–2000 |
| | | | AU | 691702 B2 | 21–05–1998 |
| | | | AU | 4083196 A | 24–04–1997 |
| | | | BR | 9600292 A | 23–12–1997 |
| | | | CA | 2166856 A1 | 18–04–1997 |
| | | | CN | 1148031 A | 23–04–1997 |
| | | | CZ | 9600201 A3 | 13–05–1998 |
| | | | DE | 69515306 D1 | 06–04–2000 |
| | | | DE | 69515306 T2 | 10–08–2000 |
| | | | EP | 0769475 A1 | 23–04–1997 |
| | | | FI | 960192 A | 18–04–1997 |
| | | | HU | 9600527 A2 | 30–06–1997 |
| | | | KR | 179410 B1 | 01–04–1999 |
| | | | MA | 23791 A1 | 01–10–1996 |
| | | | NO | 960243 A | 18–04–1997 |
| | | | NZ | 280787 A | 24–10–1997 |
| | | | PL | 313859 A1 | 28–04–1997 |
| | | | RU | 2129097 C1 | 20–04–1999 |
| | | | SG | 42867 A1 | 17–10–1997 |
| | | | US | 5948273 A | 07–09–1999 |
| | | | ZA | 9600406 A | 20–01–1997 |
| EP  0604742 | A | 06–07–1994 | US | 4942868 A | 24–07–1990 |
| | | | EP | 0604742 A1 | 06–07–1994 |
| | | | EP | 0645130 A2 | 29–03–1995 |
| | | | EP | 0645131 A2 | 29–03–1995 |
| | | | CA | 1330668 C | 12–07–1994 |
| | | | DE | 68927835 D1 | 10–04–1997 |
| | | | DE | 68927835 T2 | 17–07–1997 |
| | | | DK | 151589 A | 01–10–1989 |
| | | | EP | 0335851 A2 | 04–10–1989 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.** EP 04 01 2952

La présente annexe indique les membres de la famille de brevets relatifs aux documents brevets cités dans le rapport de
recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

12-08-2004

| Document brevet cité au rapport de recherche | | Date de publication | Membre(s) de la famille de brevet(s) | | Date de publication |
|---|---|---|---|---|---|
| EP 0604742 | A | | FI | 891461 A | 01-10-1989 |
| | | | FI | 953652 A | 31-07-1995 |
| | | | JP | 1923538 C | 25-04-1995 |
| | | | JP | 2203859 A | 13-08-1990 |
| | | | JP | 6049054 B | 29-06-1994 |
| | | | JP | 7000469 A | 06-01-1995 |
| | | | NO | 891321 A ,B, | 02-10-1989 |
| | | | US | 5048520 A | 17-09-1991 |
| | | | US | 5665141 A | 09-09-1997 |
| | | | US | 5178134 A | 12-01-1993 |
| | | | US | 5305737 A | 26-04-1994 |

EPO FORM P0460

Pour tout renseignement concernant cette annexe : voir Journal Officiel de l'Office européen des brevets, No.12/82